# EUROPEAN PATENT APPLICATION

(11) **EP 4 830 775 A1**
(43) Date of publication of application: **28.10.2026**
(21) Application number: 26172324.1
(22) Date of filing: 14.04.2026
(51) Int. Cl.: A61N 5/10

(54) **RADIATION TREATMENT PLAN OPTIMIZATION METHOD AND APPARATUS**

(30) Priority: 22.04.2025 US 202519186232
(71) Applicant: Siemens Healthineers International AG, 6312 Steinhausen (CH)
(72) Inventor: BASIRI, Shahab, 00100 Helsinki (FI); KUUSELA, Esa, 00100 Helsinki (FI)
(74) Representative: Mathisen & Macara LLP

(57) **Abstract**

To facilitate optimizing a radiation treatment plan (113) for a particular patient (104) using a particular radiation treatment apparatus (114), by one approach, a control circuit (101) iteratively accesses (202) a fluence space optimization solution for a radiation treatment plan (113) for the particular patient (104), generates (203) control point parameters for the particular radiation treatment apparatus (114) as a function of the fluence space optimization solution, determines (204) a treatment delivery time using the control point parameters for the particular radiation treatment apparatus (114), and, when treatment delivery time is suboptimal, modifies (206) the fluence space optimization solution.

## Description

### TECHNICAL FIELD

These teachings relate generally to treating a patient's planning target volume with energy pursuant to an energy-based treatment plan and more particularly to optimizing an energy-based treatment plan.

### BACKGROUND

The use of energy to treat medical conditions comprises a known area of prior art endeavor. For example, radiation therapy comprises an important component of many treatment plans for reducing or eliminating unwanted tumors. Unfortunately, applied energy does not inherently discriminate between unwanted material and adjacent tissues, organs, or the like that are desired or even critical to continued survival of the patient. As a result, energy such as radiation is ordinarily applied in a carefully administered manner to at least attempt to restrict the energy to a given target volume. A so-called radiation treatment plan often serves in the foregoing regards.

A radiation treatment plan typically comprises specified values for each of a variety of treatment-platform parameters during each of a plurality of sequential fields. Treatment plans for radiation treatment sessions are often automatically generated through a so-called optimization process. As used herein, "optimization" will be understood to refer to improving a candidate treatment plan without necessarily ensuring that the optimized result is, in fact, the singular best solution. Such optimization often includes automatically adjusting one or more physical treatment parameters (often while observing one or more corresponding limits in these regards) and mathematically calculating a likely corresponding treatment result (such as a level of dosing) to identify a given set of treatment parameters that represent a good compromise between the desired therapeutic result and avoidance of undesired collateral effects.

Radiation treatment plan optimization often consists of solving two sub-problems. First, fluence space optimization focuses on finding a dosimetrically best geometrical way of distributing the radiation. And second, control point optimization focuses on finding an efficient way of delivering this distribution (or a distribution that is close to the optimal distribution) using the intended treatment machine.

### SUMMARY OF INVENTION

In accordance with a first aspect of the invention, there is provided a method as defined by claim 1.

In accordance with a second aspect of the invention, there is provided an apparatus as defined by claim 8.

In accordance with a third aspect of the invention, there is provided a non-transitory computer-readable medium as defined by claim 15.

Optional features are defined by the dependent claims.

### BRIEF DESCRIPTION OF DRAWINGS

Various needs are at least partially met through provision of the radiation treatment plan optimization method and apparatus described in the following detailed description, particularly when studied in conjunction with the drawings, wherein:
FIG. 1 comprises a block diagram as configured in accordance with various embodiments of these teachings;
FIG. 2 comprises a flow diagram as configured in accordance with various embodiments of these teachings;
FIG. 3 comprises a flow diagram as configured in accordance with various embodiments of these teachings; and
FIG. 4 comprises a schematic representation as configured in accordance with various embodiments of these teachings.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present teachings. Also, common but well-understood elements that are useful or necessary in a commercially feasible embodiment are often not depicted in order to facilitate a less obstructed view of these various embodiments of the present teachings. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein. The word "or" when used herein shall be interpreted as having a disjunctive construction rather than a conjunctive construction unless otherwise specifically indicated.

### DETAILED DESCRIPTION

Generally speaking, these various embodiments can provide for optimizing a radiation treatment plan for a particular patient using a particular radiation treatment apparatus. By one approach, a control circuit iteratively accesses a fluence space optimization solution for a radiation treatment plan for the particular patient, generates control point parameters for the particular radiation treatment apparatus as a function of the fluence space optimization solution, determines a treatment delivery time using the control point parameters for the particular radiation treatment apparatus, and, when treatment delivery time is suboptimal, modifies the fluence space optimization solution. Optionally, when the treatment delivery time is not suboptimal, the control circuit may output the optimized radiation treatment plan corresponding to a final fluence space optimization solution.

By one approach, the aforementioned generation of the control point parameters can comprise generating a lossless conversion from input fluence information into the control point parameters such that the particular radiation treatment apparatus could, at least theoretically, deliver fluences at least nearly identical to the fluence space optimization solution. By one approach, generating the control point parameters can initially be undertaken without any limitations on how many control points are automatically allocated. In lieu of the foregoing or in combination therewith, by one approach generating the control point parameters can be initially undertaken without any limitations on how much time is required for the particular radiation treatment apparatus to accomplish the input fluence information.

By one approach, the aforementioned determination of a treatment delivery time using the control point parameters for the particular radiation treatment apparatus can include determining a time requirement for each of a plurality of different radiation treatment apparatus movement axes.

By one approach, the aforementioned modification of the fluence space optimization solution can comprise modifying the fluence space optimization solution as a function of individual time requirements for at least some of the plurality of different radiation treatment apparatus movement axes.

If desired, these teachings will also accommodate updating the fluence space optimization solution as a function of the increased compression level for the identified fluence particulars. By one approach, to provide the aforementioned increased compression level for the identified fluence particulars, these teachings will optionally provide for generating a time-requirement ordered list of the different radiation treatment apparatus movement axes for at least some control points, identifying fluence particulars that correspond to slower radiation treatment apparatus movement axes to provide identified fluence particulars, and applying an increased compression level for the identified fluence particulars. As one illustrative example, the aforementioned fluence particulars may comprise fluence rows. For example, the time-requirement ordered list may comprise a list of the different radiation treatment apparatus movement axes in order of respective time required.

If desired, these teachings will further accommodate responding to a user request by presenting information comprising a calculated dose distribution based on the increased compressed level for the identified fluence particulars.

These teachings will also accommodate optionally administering therapeutic radiation to the particular patient using the particular radiation treatment apparatus as a function of the radiation treatment plan.

By one approach, these teachings can comprise a non-transitory computer-readable medium having a computer program that itself comprises instructions that, when the computer program is executed by a computer, causes the computer to carry out any one or more of the steps, functions, and/or activities described herein including, by way of example, iteratively accessing a fluence space optimization solution for a radiation treatment plan for the particular patient, generates control point parameters for the particular radiation treatment apparatus as a function of the fluence space optimization solution, determining a treatment delivery time using the control point parameters for the particular radiation treatment apparatus, and, when treatment delivery time is suboptimal, modifying the fluence space optimization solution.

So configured, these teachings can provide an approach to radiation treatment plan optimization where the fluence space optimization and the control point optimization are solved iteratively starting from a full fluence space optimization solution and gradually reducing the fidelity requirement of the control point optimization until a solution with acceptable treatment delivery time is reached.

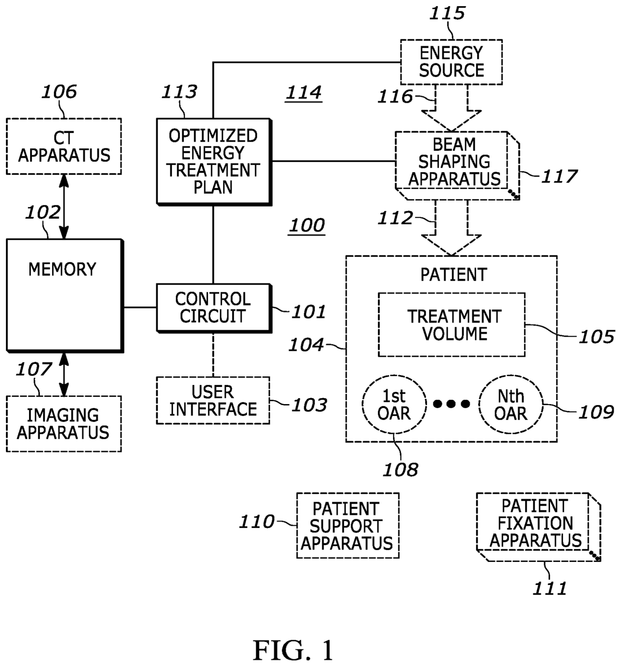

These and other benefits may become clearer upon making a thorough review and study of the following detailed description. Referring now to the drawings, and in particular to FIG. 1, an illustrative apparatus 100 that is compatible with many of these teachings will first be presented.

In this particular example, the enabling apparatus 100 includes a control circuit 101. Being a "circuit," the control circuit 101 therefore comprises structure that includes at least one (and typically many) electrically-conductive paths (such as paths comprised of a conductive metal such as copper or silver) that convey electricity in an ordered manner, which path(s) will also typically include corresponding electrical components (both passive (such as resistors and capacitors) and active (such as any of a variety of semiconductor-based devices) as appropriate) to permit the circuit to effect the control aspect of these teachings.

Such a control circuit 101 can comprise a fixed-purpose hard-wired hardware platform (including but not limited to an application-specific integrated circuit (ASIC) (which is an integrated circuit that is customized by design for a particular use, rather than intended for general-purpose use), a field-programmable gate array (FPGA), and the like) or can comprise a partially or wholly-programmable hardware platform (including but not limited to microcontrollers, microprocessors, and the like). These architectural options for such structures are well known and understood in the art and require no further description here. This control circuit 101 is configured (for example, by using corresponding programming as will be well understood by those skilled in the art) to carry out one or more of the steps, actions, and/or functions described herein.

It will be appreciated that the control circuit 101 may comprise a single integrated platform or may comprise a plurality of such circuits that work in cooperation with one another.

The control circuit 101 operably couples to a memory 102. This memory 102 may be integral to the control circuit 101 or can be physically discrete (in whole or in part) from the control circuit 101 as desired. This memory 102 can also be local with respect to the control circuit 101 (where, for example, both share a common circuit board, chassis, power supply, and/or housing) or can be partially or wholly remote with respect to the control circuit 101 (where, for example, the memory 102 is physically located in another facility, metropolitan area, or even country as compared to the control circuit 101). As with the control circuit 101, the memory 102 may comprise a singular structure or may comprise a plurality of memory platforms that collectively comprise the "memory" of this apparatus 100.

In addition to information such as optimization information for a particular patient and information regarding a particular radiation treatment platform as described herein, this memory 102 can serve, for example, to non-transitorily store the computer instructions that, when executed by the control circuit 101, cause the control circuit 101 to behave as described herein. (As used herein, this reference to "non-transitorily" will be understood to refer to a non-ephemeral state for the stored contents (and hence excludes when the stored contents merely constitute signals or waves) rather than volatility of the storage media itself and hence includes both nonvolatile memory (such as read-only memory (ROM) as well as volatile memory (such as a dynamic random access memory (DRAM).)

By one optional approach the control circuit 101 also operably couples to a user interface 103. This user interface 103 can comprise any of a variety of user-input mechanisms (such as, but not limited to, keyboards and keypads, cursor-control devices, touch-sensitive displays, speech-recognition interfaces, gesture-recognition interfaces, and so forth) and/or user-output mechanisms (such as, but not limited to, visual displays, audio transducers, printers, and so forth) to facilitate receiving information and/or instructions from a user and/or providing information to a user.

If desired the control circuit 101 can also operably couple to a network interface (not shown). So configured the control circuit 101 can communicate with other elements (both within the apparatus 100 and external thereto) via the network interface. Network interfaces, including both wireless and non-wireless platforms, are well understood in the art and require no particular elaboration here.

By one approach, a computed tomography apparatus 106 and/or other imaging apparatus 107 as are known in the art can source some or all of any desired patient-related imaging information.

In this illustrative example the control circuit 101 is configured to ultimately output an optimized energy-based treatment plan (such as, for example, an optimized radiation treatment plan 113). This energy-based treatment plan typically comprises specified values for each of a variety of treatment-platform parameters during each of a plurality of sequential exposure fields. In this case the energy-based treatment plan is generated through an optimization process, examples of which are provided further herein.

By one approach the control circuit 101 can operably couple to an energy-based treatment platform 114 that is configured to deliver therapeutic energy 112 to a corresponding patient 104 having at least one treatment volume 105 and also one or more organs-at-risk (represented in FIG. 1 by a first through an Nth organ-at-risk 108 and 109) in accordance with the optimized energy-based treatment plan 113. These teachings are generally applicable for use with any of a wide variety of energy-based treatment platforms/apparatuses. In a typical application setting the energy-based treatment platform 114 will include an energy source such as a radiation source 115 of ionizing radiation 116.

By one approach this radiation source 115 can be selectively moved via a gantry along an arcuate pathway (where the pathway encompasses, at least to some extent, the patient themselves during administration of the treatment). The arcuate pathway may comprise a complete or nearly complete circle as desired. By one approach the control circuit 101 controls the movement of the radiation source 115 along that arcuate pathway, and may accordingly control when the radiation source 115 starts moving, stops moving, accelerates, de-accelerates, and/or a velocity at which the radiation source 115 travels along the arcuate pathway.

As one illustrative example, the radiation source 115 can comprise, for example, a radio-frequency (RF) linear particle accelerator-based (linac-based) x-ray source. A linac is a type of particle accelerator that greatly increases the kinetic energy of charged subatomic particles or ions by subjecting the charged particles to a series of oscillating electric potentials along a linear beamline, which can be used to generate ionizing radiation (e.g., X-rays) 116 and high energy electrons.

A typical energy-based treatment platform 114 may also include one or more support apparatuses 110 (such as a couch) to support the patient 104 during the treatment session, one or more patient fixation apparatuses 111, a gantry or other movable mechanism to permit selective movement of the radiation source 115, and one or more energy-shaping apparatuses (for example, beam-shaping apparatuses 117 such as jaws, multi-leaf collimators, and so forth) to provide selective energy shaping and/or energy modulation as desired.

In a typical application setting, it is presumed herein that the patient support apparatus 110 is selectively controllable to move in any direction (i.e., any X, Y, or Z direction) during an energy-based treatment session by the control circuit 101. As the foregoing elements and systems are well understood in the art, further elaboration in these regards is not provided here except where otherwise relevant to the description.

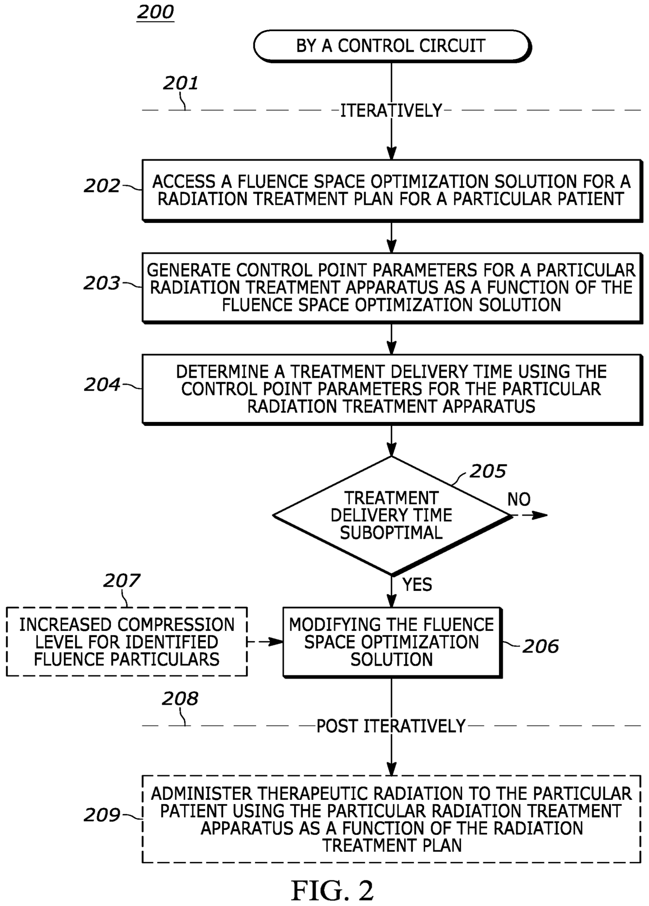

Referring now to FIG. 2, a process 200 that can be carried out, for example, in conjunction with the above-described application setting (and more particularly via the aforementioned control circuit 101) will be described. Generally speaking, this process 200 serves to facilitate generating an optimized radiation treatment plan 113 to thereby facilitate treating a particular patient with therapeutic radiation using a particular radiation treatment platform per that optimized radiation treatment plan.

As indicated by reference numeral 201, many of the steps of this process 200 occur iteratively, and in particular during iterations of the radiation treatment plan optimization process. By one approach, the described steps are undertaken during each and every optimization iteration. By another approach, the described steps are undertaken only during some iterations (for example, a first number of iterations may occur without undertaking this process 200).

At block 202, the control circuit 101 accesses a fluent space optimization solution for a radiation treatment plan for the particular patient. (Fluence represents radiative flux integrated over time and comprises a fundamental metric in dosimetry (i.e., the measurement and calculation of an absorbed dose of ionizing radiation in matter and tissue).) Accordingly, by one approach, the radiation treatment plan optimization has already begun and has progressed to the point where a fluence space optimization solution is available.

At block 203, the control circuit 101 generates control point parameters for a particular radiation treatment apparatus as a function of the accessed fluence space optimization solution. (Control points refer to specific positions or angles at which a radiation beam is turned on or off during treatment. Control points typically serve to help shape the radiation beam and to optimize the dose distribution to target the tumor while sparing surrounding healthy tissues.) By one approach, generating these control point parameters can comprise generating a lossless conversion from input fluence information into the control point parameters such that the particular radiation treatment apparatus could, at least theoretically, deliver fluences at least nearly identical to the fluence space optimization solution. (As used herein, it will be understood that the word "lossless" includes "nearly lossless," as a perfectly lossless scenario can be difficult or even impossible to achieve in a practical sense. By one approach, and as an example, "nearly lossless" could be specified as when the sum of the absolute difference between the optimal fluence and reproduced fluence is no more than 5% of the total fluence intensity.)

By one approach, generating the aforementioned control point parameters can be initially undertaken without any limitations on how many control points are automatically allocated. In lieu of the foregoing, or in combination therewith, these teachings will also accommodate generating the aforementioned control point parameters initially without any limitations on how much time is required for the particular radiation treatment apparatus to accomplish the input fluence information.

At block 204, the control circuit 101 determines a treatment delivery time using the generated control point parameters for the particular radiation treatment apparatus. By one approach, this treatment delivery time represents a start-to-finish delivery time. By another approach, if desired, the treatment delivery time can be specified in some other way. By one approach, these teachings will accommodate determining a time requirement for each of a plurality of different radiation treatment apparatus movement axes (such as different delivery positions for the radiation source, different positions for the particular patient during treatment, and so forth).

At block 205, the control circuit 101 then determines whether the aforementioned treatment delivery time is suboptimal. In a typical application setting, this determination determines whether the treatment delivery time exceeds some predetermined maximum amount of time. That maximum amount of time may be specified by the user, may be a maximum time specified by the treatment facility, may be a default value employed by the control circuit 101, or may be otherwise determined as desired.

When this determination concludes that the treatment delivery time is not suboptimal, this process can accommodate any of a variety of responses. By one approach, the optimization of the radiation treatment plan can now conclude as an acceptable control point sequence has been determined. If desired, the control points can be further pruned, for example, by removing very small apertures, and/or the control points can be re-ordered to improve delivery time. After these control points are defined, it is also possible to proceed in different ways according to the optimization scheme. This could mean, for example, terminating the optimization process and accepting the control points as such, or there could be a further optimization step (for example, one that is based on a new dose calculation with improved accuracy that has been calculated using the control points defined above).

When this determination concludes that the treatment delivery time is suboptimal, however, at block 206 the control circuit 101 modifies the fluence space optimization solution for use in a subsequent iteration cycle that again employs this process 200. By one approach, modifying the fluence space optimization solution can comprise modifying the fluence space optimization solution as a function of individual time requirements for at least some of the plurality of different radiation treatment apparatus movement axes.

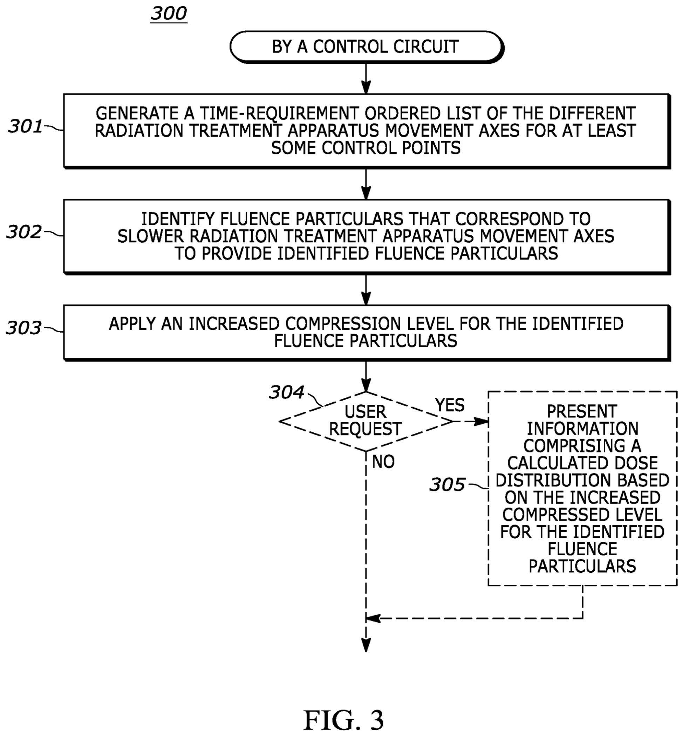

By one optional approach, modifying the fluence space optimization solution can further comprise updating the fluence space optimization solution as a function of an increased compression level for the identified fluence particulars 207. Referring momentarily to FIG. 3, an illustrative approach to provide such content will now be described.

At block 301, the control circuit 101 generates a time-requirement ordered list of the different radiation treatment apparatus movement axes for at least some control points. At block 302, the control circuit 101 then identifies fluence particulars that correspond to slower radiation treatment apparatus movement axes to provide identified fluence particulars. And at block 303, this process 300 provides for applying an increased compression level for the identified fluence particulars.

By one approach, the aforementioned fluence particulars can comprise, at least in part, fluence rows. Fluence can be represented with a fluence map, which is a gridlike representation of fluence distribution. Such a map can present the intensity levels at different points across the treatment field. Such a map is often divided into small areas called pixels or bins, and the intensity level in each bin represents the amount of radiation that should be delivered to that specific area. In that context, a "fluence row" refers to a single row of pixels or bins within that grid. A fluence row is therefore essentially a one-dimensional slice or section of the fluence map, running across the treatment field in a specified direction. Each pixel in the row indicates the intensity level of the beam that should be delivered to the corresponding section of the treatment area.

By one optional approach, and as indicated at optional block 304, this process 300 can monitor for a user request. In response to detecting that user request, and as indicated at optional block 305, the control circuit 101 can present information comprising a calculated dose distribution based on the now increased compressed level for the identified fluence particulars.

Referring again to FIG. 2, and as denoted by reference numeral 208, the iterative process described above concludes. Post iterations, with an optimized radiation treatment plan now being available, at optional block 209 this process will accommodate administering therapeutic radiation to the particular patient using the particular radiation treatment platform as a function of that optimized radiation treatment plan.

Further details that comport with these teachings will now be presented. It will be understood that the specific details of these examples are intended to serve an illustrative purpose and are not intended to suggest any particular limitations with respect to these teachings.

It may first be helpful to some readers to have additional information regarding current optimization techniques.

Fluence space optimization typically includes finding a set of fluence maps, whose does distribution minimizes a given cost function, formulated based on the plan objectives. This is often a relatively well-behaving problem, and can be efficiently solved using standard gradient-based methods. To deliver the fluence space optimization solution using the radiation treatment platform, the fluence map(s) need to be converted to control point parameters such as multi-leaf collimator leaf positions, monitor units, and so forth.

In control point optimization, typically a previously-defined number of control points are optimized to deliver a dose distribution that is close to the fluence space optimization solution with possibly additional cost terms related to treatment time (either directly or indirectly) and/or other parameters associated with the treatment complexity (such as aperture shape constraints). In practice, control point optimization typically aims at maximizing fidelity of the dose delivery (for example, by minimizing the conversion loss) as constrained by the speed limits that apply to the moving axes of the machine.

Control point optimization is typically non-convex and has high degrees of freedom. The variations of the gradient methods used to address the problem are sensitive to initialization and the applicant has determined that such approaches may easily get stuck into suboptimal solutions. Moreover, due to performance reasons, the control point optimization methods often use crude approximations of the first and second order gradients leaving the validity of the solutions questionable. Consequently, the applicant has determined that machine-generated fluences can deviate significantly from the optimal fluence space optimization solutions. This can result in suboptimal dose distributions and degraded plan quality.

The optimal achievable plan quality for a given set of plan objectives is usually not revealed to the planner since the fluence space optimization solutions are typically only internal to the radiation treatment planning optimization algorithms. Even if such a benchmark is provided, improving the performance of the control point optimization methods for each specific case requires complex additional tools and interfaces to enable the planner to manually allocate more control points to certain directions or relax the speed limits by extending the delivery time. In lieu of the foregoing or in combination therewith, the planner may also use case sensitive initialization methods or choose among various control point optimization solver algorithms. The applicant has determined that dealing with such tools makes the plan generation process even more tedious and time consuming.

Given the foregoing context, typical existing methods find only suboptimal solutions to the control point optimization problem by using either approximate gradient methods or random search policies with a large number of trials. This implies that the conversion from fluence space optimization fluence maps to control point parameters can be very lossy. Nevertheless, the infidelity of the conversion is typically simply neglected. Instead, the radiation treatment planning optimization continues without knowing how much better plan quality could be achieved if the conversion loss was under regulation.

To address at least some of the foregoing concerns, these teachings can provide an approach to radiation treatment planning optimization where the described conversion is not formulated as an optimization problem. For example, the conversion from fluence space optimization fluence maps to control point parameters may not be formulated as an optimization problem. As a result, solving control point optimization as a non-convex high-dimensional problem is avoided. Instead, the process starting with the fluence space optimization solution, generating an optimal quality plan by lossless control point creation, and then gradually increasing the fluence compression until the desired level of plan complexity (or treatment delivery time) is achieved.

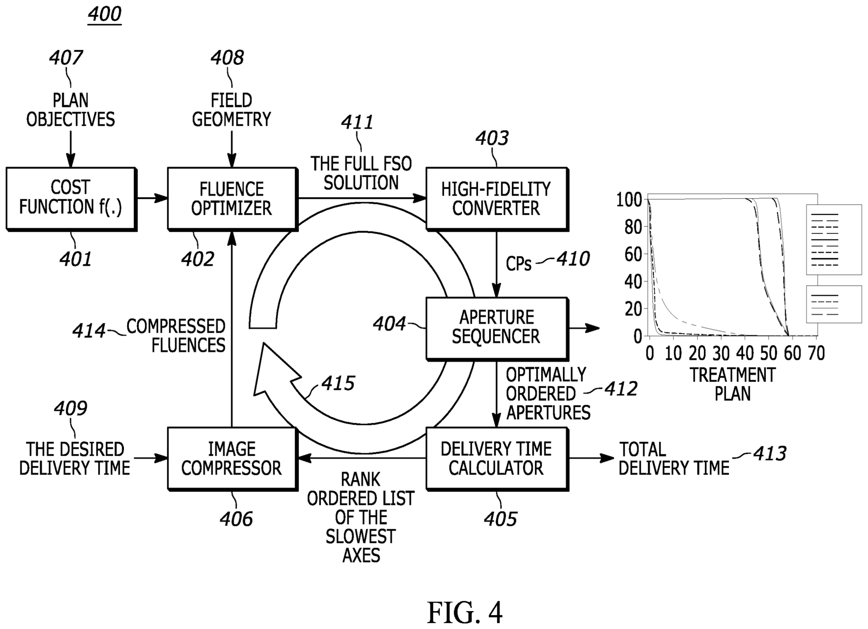

FIG. 4 presents an illustrative example in these regards. It will be understood that the details of this example are intended to serve an illustrative purpose and are not intended to suggest any limitations with respect to these teachings.

In addition to a cost function 401 that is based on plan objectives and a fluence optimizer 402 that operates on cost function 401 and/or the corresponding field geometry of the application setting, this illustrative apparatus 400 also includes a high-fidelity converter 403, an aperture sequencer 404, a delivery time calculator 405, and an image compressor 406. These components are optional and one or more of the high-fidelity converter 403, the aperture sequencer 404, the delivery time calculator 405, and the image compressor 406 may be dispensed with.

The high-fidelity converter 403 performs lossless conversion, or near lossless conversion, from the input fluence maps into control point parameters such that the treatment machine can deliver fluences that are at least nearly identical to the input. Note that here there is no constraint on the time it takes for the machine to generate those fluences. In addition, the required number of control points (e.g. a count of the number of control points) is neither set nor restricted. Instead, the high-fidelity converter 403 may allocate as many control points as needed to facilitate a lossless conversion. The number of required control points may depend, for example, on the complexity of the input fluences. Such converters sometimes use variations of the sliding window techniques and these teachings will accommodate use of such a converter. For example, a sliding window technique may be used to convert the input fluence maps to control point parameters.

The aperture sequencer 404 finds a reasonable or optimal rank order of the multi-leaf collimator apertures such that the leaves in neighboring control points do not waste too much of the delivery time for unnecessarily moving back and forth. (If desired, these teachings will support including this sequencing activity in the high-fidelity converter 403).

The delivery time calculator 405 is configured to calculate the time it takes for the treatment machine to treat a given sequence of control points. As part of calculating the total time, the delivery time calculator 405 can also be configured to provide detailed timing information for each moving axes of the treatment machine.

The image compressor 406 can comprise a conventional adjustable compressor used in image processing to reduce the size of the input fluence rows to a desired level. These teachings will accommodate use of a variety of approaches in these regards, ranging from methods that are based on traditional wavelet decomposition to more advanced methods that leverage use of a neural network or other form of artificial intelligence.

Presuming use of the foregoing components, an illustrative use case for optimizing a radiation treatment plan will now be provided.

At the outset, the user/planner can set a desired delivery time T₀ 409. A fluence space optimization solution 411 is then obtained by running the fluence optimizer 402 for a given set of plan objectives 407 as well as field geometries 408 relevant to the treatment setting. The image compressor 406 can be set to an initial compression level of zero.

The high-fidelity converter 403 outputs a determined number of control points 410 as well as the control point parameters required for the lossless delivery of the fluence space optimization fluences. For example, the high-fidelity converter 403 may specify a count of the number of control points. The aperture sequencer 404 outputs a ranked order 412 for the apertures and the delivery time calculator 405 outputs the total delivery time Tₜ 413 of the benchmark plan.

Presuming the total delivery time Tₜ 413 is greater than the desired delivery time T₀ 409, one can then identify the fluence rows (or the full map, e.g. the full fluence map) that correspond to the slowest axes, and for the fluence rows (or the full map, e.g. the full fluence map) that are causing (or most responsible for causing) slowdowns, increase the compression level in the image compressor 406 by a step size ε, which step size can then be applied as a level of compression in the image compressor 406 to the fluences.

The compressed fluences 414 are provided to the fluence optimizer which updates the previous fluence space optimization solution for the uncompressed fluences. The high-fidelity converter 403 updates the control points 410 and the required parameters for the lossless delivery of the new solution and the aperture sequencer 404 outputs a new rank order for the output apertures.

The delivery time calculator 405 recalculates the total delivery time Tₜ 413 and the appropriate fluences are now compressed as before and a new fluence space optimization solution is provided.

This iterative approach can continuously cycle as described (as indicated by the arrow that is denoted by reference numeral 415) until the total delivery time is equal to or less than the desired delivery time. (These teachings will also accommodate halting the process if, for example, a given number of iteration cycles is reached.)

If desired, and upon detecting a user's/planner's request for intermediate plan information, these teachings will accommodate calculating the dose distribution that corresponds to the compressed fluences, for example intermediate dose distributions, and presenting that information via a user interface.

If desired, these teachings will accommodate using the control point specific times calculated by the delivery time calculator 405 to train a reinforcement learning agent to apply a fluence-specific compression level. Using this approach, it may be possible to minimize the effect of compression to the plan quality.

And again if desired, these teachings, for example the apparatus, could be configured to provide to a user/planner a desired or given number of plan alternatives (e.g. a plurality of plan alternatives) that range from a best-quality but slow-to-deliver plan to a degraded-quality but fastest-to-deliver plan. The user/planner could then choose a given plan that best suited the needs of a given application setting. For example, the user/planner may select a plan with a desired trade-off between quality and speed.

These teachings can help to minimize the time the optimizer spends in searching the large non-convex solution space of the control point optimization problem. These teachings can also help minimize the time the user/planner may spend searching unfruitful parts of the solution space. Unlike typical existing methods, these teachings provide the optimal fluence space optimization solution as well as the corresponding delivery time as the benchmark for a given cost function. In addition, these teachings can enable the user/planner to make informed decision with respect to trading off between plan quality and delivery time. Those skilled in the art will also appreciate that these teachings are graphics processing unit (GPU) friendly and tend to be fast to compute, and also can readily interact with artificial intelligence technology to off-load the plan generation task from the human user/planner.

Further aspects of these teachings are provided by the subject matter of the following clauses (where it will be understood that any of these clauses can be combined with any one or more of the other clauses as desired).

Clause 1. A method for optimizing a radiation treatment plan for a particular patient using a particular radiation treatment apparatus, the method comprising: by a control circuit: iteratively: accessing a fluence space optimization solution for a radiation treatment plan for the particular patient; generating control point parameters for the particular radiation treatment apparatus as a function of the fluence space optimization solution; determining a treatment delivery time using the control point parameters for the particular radiation treatment apparatus; when treatment delivery time is suboptimal, modifying the fluence space optimization solution.

Clause 2. The method of clause 1 wherein generating the control point parameters comprises generating a lossless conversion from input fluence information into the control point parameters such that the particular radiation treatment apparatus could, at least theoretically, deliver fluences at least nearly identical to the fluence space optimization solution.

Clause 3. The method of clause 1 or clause 2 wherein generating the control point parameters is initially undertaken without any limitations on how many control points are automatically allocated.

Clause 4. The method of any preceding clause wherein generating the control point parameters is initially undertaken without any limitations on how much time is required for the particular radiation treatment apparatus to accomplish the input fluence information.

Clause 5. The method of any preceding clause wherein determining a treatment delivery time using the control point parameters for the particular radiation treatment apparatus includes determining a time requirement for each of a plurality of different radiation treatment apparatus movement axes.

Clause 6. The method of clause 5 wherein modifying the fluence space optimization solution comprises modifying the fluence space optimization solution as a function of individual time requirements for at least some of the plurality of different radiation treatment apparatus movement axes.

Clause 7. The method of clause 5 or clause 6 further comprising: generating a time-requirement ordered list of the different radiation treatment apparatus movement axes for at least some control points; identifying fluence particulars that correspond to slower radiation treatment apparatus movement axes to provide identified fluence particulars; applying an increased compression level for the identified fluence particulars; and wherein modifying the fluence space optimization solution comprises updating the fluence space optimization solution as a function of the increased compression level for the identified fluence particulars.

Clause 8. The method of clause 7 wherein the fluence particulars comprise fluence rows.

Clause 9. The method of clause 7 or clause 8 further comprising: in response to a user request, present information comprising a calculated dose distribution based on the increased compressed level for the identified fluence particulars.

Optional Clause 10. The method of any preceding clause further comprising: administering therapeutic radiation to the particular patient using the particular radiation treatment apparatus as a function of the radiation treatment plan.

Clause 11. An apparatus for optimizing a radiation treatment plan for a particular patient using a particular radiation treatment apparatus, the apparatus comprising: a control circuit configured to iteratively: access a fluence space optimization solution for a radiation treatment plan for the particular patient; generate control point parameters for the particular radiation treatment apparatus as a function of the fluence space optimization solution; determine a treatment delivery time using the control point parameters for the particular radiation treatment apparatus; when treatment delivery time is suboptimal, modify the fluence space optimization solution.

Clause 12. The apparatus of clause 11 wherein the control circuit is configured to generate the control point parameters by generating a lossless conversion from input fluence information into the control point parameters such that the particular radiation treatment apparatus could, at least theoretically, deliver fluences at least nearly identical to the fluence space optimization solution.

Clause 13. The apparatus of clause 11 of clause 12 wherein the control circuit is configured to generate the control point parameters by initially generating the control point parameters without any limitations on how many control points are automatically allocated.

Clause 14. The apparatus of any of clause 11 to clause 13 wherein the control circuit is configured to generate the control point parameters by initially generating the control point parameters without any limitations on how much time is required for the particular radiation treatment apparatus to accomplish the input fluence information.

Clause 15. The apparatus of any of clause 11 to clause 14 wherein the control circuit is configured to determine a treatment delivery time using the control point parameters for the particular radiation treatment apparatus by determining a time requirement for each of a plurality of different radiation treatment apparatus movement axes.

Clause 16. The apparatus of clause 15 wherein the control circuit is configured to modify the fluence space optimization solution by modifying the fluence space optimization solution as a function of individual time requirements for at least some of the plurality of different radiation treatment apparatus movement axes.

Clause 17. The apparatus of clause 15 or clause 16 wherein the control circuit is further configured to: generate a time-requirement ordered list of the different radiation treatment apparatus movement axes for at least some control points; identify fluence particulars that correspond to slower radiation treatment apparatus movement axes to provide identified fluence particulars; apply an increased compression level for the identified fluence particulars; such that modifying the fluence space optimization solution comprises updating the fluence space optimization solution as a function of the increased compression level for the identified fluence particulars.

Clause 18. The apparatus of clause 17 wherein the fluence particulars comprise fluence rows.

Clause 19. The apparatus of clause 17 or clause 18 wherein the control circuit is further configured to: in response to a user request, present information comprising a calculated dose distribution based on the increased compressed level for the identified fluence particulars.

Clause 19A. The apparatus of any of clause 11 to clause 19 further configured to administer therapeutic radiation to the particular patient using the particular radiation treatment apparatus as a function of the radiation treatment plan.

Clause 20. A non-transitory computer-readable medium for optimizing a radiation treatment plan for a particular patient using a particular radiation treatment apparatus comprising instructions stored thereon, that when executed on a processor, perform the steps of: iteratively: accessing a fluence space optimization solution for a radiation treatment plan for the particular patient; generating control point parameters for the particular radiation treatment apparatus as a function of the fluence space optimization solution; determining a treatment delivery time using the control point parameters for the particular radiation treatment apparatus; when treatment delivery time is suboptimal, modifying the fluence space optimization solution.

Those skilled in the art will recognize that a wide variety of modifications, alterations, and combinations can be made with respect to the above described embodiments without departing from the scope of the invention, and that such modifications, alterations, and combinations are to be viewed as being within the ambit of the inventive concept.

## Claims

1. A method for optimizing a radiation treatment plan for a particular patient using a particular radiation treatment apparatus, the method comprising:
by a control circuit:
iteratively:
accessing a fluence space optimization solution for a radiation treatment plan for the particular patient;
generating control point parameters for the particular radiation treatment apparatus as a function of the fluence space optimization solution;
determining a treatment delivery time using the control point parameters for the particular radiation treatment apparatus;
when treatment delivery time is suboptimal, modifying the fluence space optimization solution.

2. The method of claim 1 wherein generating the control point parameters comprises generating a lossless conversion from input fluence information into the control point parameters such that the particular radiation treatment apparatus could, at least theoretically, deliver fluences at least nearly identical to the fluence space optimization solution.

3. The method of claim 1 or claim 2 wherein generating the control point parameters is initially undertaken without any limitations on how many control points are automatically allocated;
and/or:
wherein generating the control point parameters is initially undertaken without any limitations on how much time is required for the particular radiation treatment apparatus to accomplish the input fluence information.

4. The method of any preceding claim wherein determining a treatment delivery time using the control point parameters for the particular radiation treatment apparatus includes determining a time requirement for each of a plurality of different radiation treatment apparatus movement axes.

5. The method of claim 4 wherein modifying the fluence space optimization solution comprises modifying the fluence space optimization solution as a function of individual time requirements for at least some of the plurality of different radiation treatment apparatus movement axes.

6. The method of claim 4 or claim 5 further comprising:
generating a time-requirement ordered list of the different radiation treatment apparatus movement axes for at least some control points;
identifying fluence particulars that correspond to slower radiation treatment apparatus movement axes to provide identified fluence particulars;
applying an increased compression level for the identified fluence particulars; and wherein modifying the fluence space optimization solution comprises updating the fluence space optimization solution as a function of the increased compression level for the identified fluence particulars;
and optionally:
wherein the fluence particulars comprise fluence rows.

7. The method of claim 6 further comprising:
in response to a user request, present information comprising a calculated dose distribution based on the increased compressed level for the identified fluence particulars.

8. An apparatus for optimizing a radiation treatment plan for a particular patient using a particular radiation treatment apparatus, the apparatus comprising:
a control circuit configured to iteratively:
access a fluence space optimization solution for a radiation treatment plan for the particular patient;
generate control point parameters for the particular radiation treatment apparatus as a function of the fluence space optimization solution;
determine a treatment delivery time using the control point parameters for the particular radiation treatment apparatus;
when treatment delivery time is suboptimal, modify the fluence space optimization solution.

9. The apparatus of claim 8 wherein the control circuit is configured to generate the control point parameters by generating a lossless conversion from input fluence information into the control point parameters such that the particular radiation treatment apparatus could, at least theoretically, deliver fluences at least nearly identical to the fluence space optimization solution.

10. The apparatus of claim 8 or claim 9 wherein the control circuit is configured to generate the control point parameters by initially generating the control point parameters without any limitations on how many control points are automatically allocated;
and/or:
wherein the control circuit is configured to generate the control point parameters by initially generating the control point parameters without any limitations on how much time is required for the particular radiation treatment apparatus to accomplish the input fluence information.

11. The apparatus of any of claim 8 to claim 10 wherein the control circuit is configured to determine a treatment delivery time using the control point parameters for the particular radiation treatment apparatus by determining a time requirement for each of a plurality of different radiation treatment apparatus movement axes.

12. The apparatus of claim 11 wherein the control circuit is configured to modify the fluence space optimization solution by modifying the fluence space optimization solution as a function of individual time requirements for at least some of the plurality of different radiation treatment apparatus movement axes.

13. The apparatus of claim 11 or claim 12 wherein the control circuit is further configured to:
generate a time-requirement ordered list of the different radiation treatment apparatus movement axes for at least some control points;
identify fluence particulars that correspond to slower radiation treatment apparatus movement axes to provide identified fluence particulars;
apply an increased compression level for the identified fluence particulars;
such that modifying the fluence space optimization solution comprises updating the fluence space optimization solution as a function of the increased compression level for the identified fluence particulars;
and optionally:
wherein the fluence particulars comprise fluence rows.

14. The apparatus of claim 13 wherein the control circuit is further configured to:
in response to a user request, present information comprising a calculated dose distribution based on the increased compressed level for the identified fluence particulars.

15. A non-transitory computer-readable medium for optimizing a radiation treatment plan for a particular patient using a particular radiation treatment apparatus comprising instructions stored thereon, that when executed on a processor, perform the steps of:
iteratively:
accessing a fluence space optimization solution for a radiation treatment plan for the particular patient;
generating control point parameters for the particular radiation treatment apparatus as a function of the fluence space optimization solution;
determining a treatment delivery time using the control point parameters for the particular radiation treatment apparatus;
when treatment delivery time is suboptimal, modifying the fluence space optimization solution;
and optionally wherein:
the instructions, when executed on the processor, perform the method of any of claim 2 to claim 7.
